# FASCICULE DE BREVET EUROPEEN

(11) **EP 2 411 135 B1**
(45) Date de publication et mention de la délivrance du brevet: **25.09.2013**
(21) Numéro de dépôt: 10715974.1
(22) Date de dépôt: 24.03.2010
(51) Int. Cl.: B01F 13/08

(54) **BIOREACTEUR JETABLE ET SYSTEME D'AGITATION A USAGE UNIQUE**
WEGWERF-BIOREAKTOR UND RÜHRSYSTEM ZUR EINMALIGEN VERWENDUNG
DISPOSABLE BIOREACTOR AND USE-ONCE STIRRING SYSTEM

(30) Priorité: 24.03.2009 FR 0951895
(43) Date de publication de la demande: 01.02.2012
(73) Titulaire: Zambaux, Jean-Pascal, 33980 Audenge (FR)
(72) Inventeur: Zambaux, Jean-Pascal, 33980 Audenge (FR)
(74) Mandataire: Hubert, Philippe
(86) Numéro de dépôt international: PCT/FR2010/050534
(87) Numéro de publication internationale: WO 2010/109136

(56) Documents cités:
- EP-A2- 1 748 201
- WO-A1-2007/050971
- WO-A1-2008/040569
- WO-A1-2008/103857
- WO-A2-2005/118771
- DE-B3-102006 022 651

## Description

La présente invention concerne un bioréacteur jetable, ainsi qu'un système d'agitation à usage unique.

### ETAT DE LA TECHNIQUE

Un bioréacteur comprend des réacteurs mis en oeuvre dans des fermentations, des réactions enzymatiques, des cultures cellulaires, ou encore utilisés dans le domaine du génie tissulaire, dans la fabrication de produits biologiques ou chimiques, de médicaments biologiques et de microorganismes. La fabrication de ces produits requiert des étapes de nettoyage, de stérilisation et de validation du matériel, tels que les bioréacteurs ou les systèmes d'agitation, extrêmement rigoureuses et contraignantes, étapes qui augmentent sensiblement le coût de fabrication de ces produits. Des bioréacteurs ainsi que des systèmes d'agitation stériles et jetables ont par conséquent été développés pour remédier à ce problème, en particulier dans le but de minimiser les temps de nettoyage et de validation et/ou d'éviter les risques de contamination. Un tel bioréacteur est décrit dans DE 10 2006 022 651 B3.

Cependant, les systèmes d'agitation à usage unique actuellement disponibles sur le marché présentent l'inconvénient de convenir seulement à un type de bioréacteur jetable donné. L'utilisateur d'un bioréacteur jetable donné est donc contraint à acquérir un système d'agitation à usage unique du fabricant commercialisant ledit bioréacteur jetable donné souvent à un prix relativement élevé.

Ainsi, il existe un réel besoin de proposer un bioréacteur jetable qui représenterait une solution alternative aux bioréacteurs existants, ainsi qu'un système d'agitation à usage unique qui ne présente pas l'inconvénient précité.

### BUTS DE L'INVENTION

L'invention a pour but principal de proposer un bioréacteur jetable de conception simple, ainsi qu'un système d'agitation à usage unique qui résout le problème précité, c'est-à-dire qui s'adapte à n'importe quel bioréacteur jetable.

Le bioréacteur jetable ainsi que le système d'agitation à usage unique selon la présente invention doivent présenter l'avantage d'être utilisable à l'échelle industrielle et commerciale, de manière sûre et fiable et leur structure simple doit permettre de fabriquer un bioréacteur jetable ainsi qu'un système d'agitation à usage unique peu coûteux.

### RESUME DE L'INVENTION

Selon son premier objet, l'invention concerne un bioréacteur jetable comprenant une poche flexible réalisée dans un matériau polymérique. Le bioréacteur comprend en outre un système d'agitation à usage unique placé en intégralité au sein de ladite poche ; ledit système d'agitation comprenant un moyen d'entrainement et au moins un moyen d'agitation entrainé en rotation par ledit moyen d'entrainement via un couplage magnétique.

En d'autres termes, ledit moyen d'entrainement et ledit au moins un moyen d'agitation du système d'agitation sont complètement disposés à l'intérieur de la poche flexible réalisée dans un matériau polymérique. Le matériau polymérique est avantageusement choisi parmi le groupe constitué par le polyéthylène, le polypropylène, les polyamides, le polyétheréthercétone (PEEK), le copolymère d'éthylène et d'alcool vinylique (EVOH) et tout plastique comportant un revêtement de surface bicouche, ladite bicouche comprenant une couche primaire interne d'hydroxypropylméthylcellulose (HPMC), ou d'alcool polyvinylique (PVA), et une couche bioactive externe de carboxyméthylcellulose située sur ladite couche interne. Le revêtement bicouche constitue une barrière entre le plastique constituant la poche et le milieu à mélanger dans la poche (avantageusement un milieu de culture cellulaire) et empêche la fixation des protéines (avantageusement des cellules) sur le plastique. La nature de ce revêtement bicouche, ainsi que son utilisation, sont plus particulièrement décrites dans le brevet FR 2 862 979 B1.

De manière générale, on entend par le terme « jetable » que le bioréacteur est stérile avant toute utilisation et qu'on s'en débarrasse après usage. L'expression « à usage unique » doit être comprise comme indiquant que le système d'agitation est stérile avant son utilisation et que celui-ci peut être employé à plusieurs reprises avant d'être jeté, tant que les réactions dans lesquelles il intervient pour mélanger le milieu se déroulent dans des conditions identiques (même réactifs, même quantité de réactifs, même solvant...).

Selon un mode de réalisation avantageux de l'invention, le système d'agitation comprend en outre un moyen d'actionnement du moyen d'entrainement constitué par une arrivée de fluide ou d'électricité (charges électriques).

Selon une variante de réalisation particulièrement avantageuse de l'invention, le fluide est un gaz choisi parmi le groupe constitué par l'air, l'azote, le dioxyde de carbone, l'argon, le dioxygène, et un mélange quelconque de ceux-ci. L'emploi du dioxyde de carbone, du dioxygène, de l'azote ou de l'air en tant que fluide est avantageusement préféré. En effet, chacun de ces gaz peut également être utilisé pour aérer le milieu à mélanger dans le bioréacteur à l'aide d'un aérateur et donc servir d'élément de nutrition aux microorganismes en croissance.

Selon un mode de réalisation particulièrement avantageux de l'invention, le bioréacteur jetable comprend en outre au moins un tuyau traversant la paroi de la poche. Avantageusement, ledit au moins un tuyau est attaché ou vient se connecter au système d'agitation au niveau du moyen d'entrainement. De manière particulièrement préférée, ledit au moins un tuyau constitue ledit moyen d'actionnement du moyen d'entrainement.

Selon un mode de réalisation avantageux de l'invention, ledit au moins un moyen d'agitation comprend un axe de rotation, ledit axe de rotation présentant au moins une surface métallique susceptible d'être attirée par une surface aimantée, de préférence un aimant, et/ou ledit axe de rotation présentant au moins une surface aimantée, de préférence au moins un aimant. Avantageusement, l'axe de rotation comporte soit une surface métallique ou une surface aimantée sensiblement sur l'ensemble de sa surface, soit une alternance de surfaces métalliques et de surfaces aimantées.

Selon un autre mode de réalisation avantageux de l'invention, ledit au moins un moyen d'agitation comprend au moins une hélice présentant un moyeu et au moins une pale, et de préférence deux hélices. Avantageusement, ladite au moins une hélice comprend plusieurs pales de différentes formes possibles. Dans le cas où ledit au moins un moyen d'agitation présente plusieurs hélices, chacune des hélices peut comporter un nombre différent de pales, éventuellement de formes différentes.

Selon encore un autre mode de réalisation avantageux de l'invention, ledit moyen d'entrainement est une turbine qui comprend des ailettes faisant partie intégrante d'un arbre creux, ledit axe de rotation dudit au moins un moyen d'agitation étant placé dans ledit creux de l'arbre et la paroi interne dudit arbre creux présentant :
- au moins une surface métallique attirée par ladite surface aimantée de l'axe de rotation dudit au moins un moyen d'agitation ; et/ou
- au moins une surface aimantée, de préférence au moins un aimant, attirant ladite au moins une surface métallique ou aimantée de l'axe de rotation dudit au moins un moyen d'agitation.

Ainsi, la paroi interne de l'arbre creux est avantageusement munie soit d'une ou de plusieurs surface(s) aimantée(s) coopérant avec une ou plusieurs surface(s) métallique(s) ou aimantée(s) de l'axe de rotation, soit d'une ou de plusieurs surface(s) métallique(s) coopérant avec une ou plusieurs surface(s) aimantée(s) de l'axe de rotation. De manière particulièrement avantageuse, lorsque l'axe de rotation comporte une surface métallique sensiblement sur l'ensemble de sa surface, la paroi interne de l'arbre creux présente au moins une surface aimantée coopérant avec cette surface métallique de l'axe de rotation ; ou bien lorsque l'axe de rotation comporte une surface aimantée de pôle A sensiblement sur l'ensemble de sa surface, la paroi interne de l'arbre creux présente au moins une surface métallique ou au moins une surface aimantée de pôle opposé au pôle A pour coopérer avec la surface aimantée de l'axe de rotation de manière à transmettre le mouvement de rotation lorsque le moyen d'entrainement est mis en rotation.

De préférence, ladite au moins une surface métallique ou ladite au moins une surface aimantée de la paroi interne de l'arbre creux est positionnée sur ou dans la paroi interne dudit arbre creux et ce, dans le prolongement de l'emplacement des ailettes.

Selon un mode de réalisation particulièrement avantageux de l'invention, la turbine comprend en outre un corps fixe ayant sensiblement la forme d'un cylindre fermé, ledit corps fixe formant une enceinte dans laquelle les ailettes sont susceptibles de se déplacer. On entend par l'expression « ayant sensiblement la forme d'un cylindre fermé », un cylindre fermé perforé au centre des disques le constituant, soit sur toute sa hauteur, soit partiellement c'est-à-dire qu'un de ces deux disques n'est pas perforé. L'espace défini par cette perforation est avantageusement de forme cylindrique et permet de loger l'axe de rotation du moyen d'agitation.

Selon un autre mode de réalisation particulièrement avantageux de l'invention, au moins une desdites ailettes est munie d'au moins un aimant coopérant avec au moins un contre-aimant positionné sur la surface inférieure dudit au moins un moyen d'agitation pour empêcher le frottement entre le moyen d'entrainement et ledit au moins un moyen d'agitation, ladite surface inférieure correspondant à la surface dudit au moins un moyen d'agitation opposée à la surface où ladite au moins une pale dudit au moins un moyen d'agitation fait saillie. Ledit au moins un aimant est, de préférence, positionné sur une ou plusieurs ailette(s) dans une région périphérique interne, c'est-à-dire proche de l'axe de rotation de l'arbre creux, mais n'est pas situé sur ou dans la paroi de l'arbre creux.

Selon une variante de réalisation avantageuse de l'invention, le corps fixe de la turbine, sous forme de cylindre, est muni d'un ou de plusieurs aimant(s) dans la région périphérique externe de la surface du ou des disque(s) dudit cylindre. De manière particulièrement avantageuse, ce ou ces aimants a(ont) une polarité identique audit au moins un contre-aimant du moyen d'agitation afin d'empêcher le frottement entre le moyen d'entrainement et ledit au moins un moyen d'agitation. De préférence, ce ou ces aimants a(ont) également une polarité identique à l'aimant ou les aimants positionné(s) sur les ailettes pour repousser plus efficacement la surface inférieure dudit au moins un moyen d'agitation.

Selon encore un autre mode de réalisation particulièrement avantageux de l'invention, ladite arrivée de fluide sert également d'aérateur du milieu à mélanger dans la poche. Ce mode de réalisation est particulièrement préféré lorsque le moyen d'actionnement du moyen d'entrainement est un gaz. La présence de l'aérateur est d'autant plus intéressante lorsque le gaz choisi contribue à la croissance de microorganismes.

Selon un mode de réalisation avantageux de l'invention, la hauteur dudit système d'agitation à usage unique dans la poche est variable et peut être variée pendant l'agitation. Le mélange du milieu réactionnel peut par conséquent être réalisé de manière homogène et peut permettre d'optimiser le rendement.

Selon une variante de réalisation avantageuse de l'invention, la hauteur du système d'agitation à usage unique dans la poche peut être modifiée grâce à la présence de moyen de fixation du système d'agitation lorsque le système d'agitation est en cours d'utilisation ou lorsqu'il ne fonctionne pas.

Le système d'agitation à usage unique peut donc être maintenu de manière stabilisée à la poche flexible du bioréacteur jetable à l'aide d'un moyen de fixation.

Selon une variante de réalisation particulièrement avantageuse de l'invention, le moyen de fixation permet de raccorder des tuyaux d'arrivée de fluide ou d'électricité en haut de la poche. Dans ce cas, le moyen de fixation est constitué d'une pièce mâle et d'une pièce femelle raccordées par un joint métallique. La pièce femelle en matière plastique est soudée à la poche. La matière plastique peut avantageusement être choisie parmi le groupe constitué par le polyéthylène, le polypropylène, les polyamides, le polyétheréthercétone (PEEK) et le copolymère d'éthylène et d'alcool vinylique (EVOH). La pièce mâle est munie de deux trous dans lesquels les tuyaux d'arrivée de fluide ou d'électricité sont placés de manière sensiblement fixe (la hauteur des tuyaux peut toutefois être modifiée sous l'action d'une force physique), et est placée sur la pièce femelle. Le joint métallique de raccordement, composé de deux demi-cercles pouvant pivoter selon un axe commun et d'une fermeture à vis, permet d'unir les pièces femelle et mâle en un bloc, le bloc étant solidaire à la poche flexible. Les tuyaux d'arrivée de fluide ou d'électricité peuvent parfois être placés dans des barres de renforcement pour les rendre plus rigides et solides. Les tuyaux d'arrivée de fluide ou d'éléctricité sont avantageusement maintenus à un support fixe à l'aide d'un système pince-noix. De manière particulièrement préférée, les tuyaux sont des tuyaux d'arrivée de fluide.

Selon une autre variante de réalisation particulièrement avantageuse de l'invention, le moyen de fixation permet de raccorder des tuyaux d'arrivée de fluide ou d'électricité à un côté de la poche. Le moyen de fixation est constitué d'un raccord double pour tuyaux. Ce raccord a la forme de deux raccords pour tuyaux d'arrosage en série, c'est-à-dire qu'il est essentiellement constitué de deux pièces mâles de forme tubulaire ayant chacun une extrémité filetée. Ces pièces mâles comprennent en outre perpendiculairement à leur tube une surface sensiblement plane, en matière plastique, permettant au raccord double d'être soudé à la poche. La matière plastique de la surface sensiblement plane peut avantageusement être choisie parmi le groupe constitué par le polyéthylène, le polypropylène, les polyamides, le polyétheréthercétone (PEEK) et le copolymère d'éthylène et d'alcool vinylique (EVOH). La forme filetée des extrémités permet de connecter les tuyaux d'arrivée de fluide ou d'électricité de manière étanche. De manière particulièrement préférée, les tuyaux sont des tuyaux d'arrivée de fluide. Des barres de renforcement peuvent venir se connecter aux extrémités non filetées et être maintenues à un support fixe à l'aide d'un système pince-noix.

Grâce à ces divers moyens de fixation, le système d'agitation peut être fixé de manière non permanente dans une poche et/ou être retiré d'une poche après utilisation pour être fixé dans une autre poche. En d'autre termes, le système d'agitation s'adapte à et/ou peut convenir à n'importe quel bioréacteur jetable par l'intermédiaire de ces moyens de fixations.

Selon son deuxième objet, l'invention concerne un système d'agitation à usage unique comprenant :
- un moyen d'entrainement, et
- au moins un moyen d'agitation entrainé en rotation par ledit moyen d'entraînement via un couplage magnétique,
ledit au moins un moyen d'agitation comprenant un axe de rotation. Ledit axe de rotation présente au moins une surface métallique susceptible d'être attirée par une surface aimantée, de préférence un aimant, et/ou ledit axe de rotation présente au moins une surface aimantée, de préférence au moins un aimant. Ledit moyen d'entraînement est une turbine qui comprend des ailettes faisant partie intégrante d'un arbre creux. L'axe de rotation dudit au moins un moyen d'agitation est placé dans ledit creux de l'arbre et la paroi interne dudit arbre creux présente :
- au moins une surface métallique attirée par ladite surface aimantée de l'axe de rotation dudit au moins un moyen d'agitation ; et/ou
- au moins une surface aimantée, de préférence au moins un aimant, attirant ladite au moins une surface métallique ou aimantée de l'axe de rotation dudit au moins un moyen d'agitation.

Avantageusement, l'axe de rotation comporte soit une surface métallique ou une surface aimantée sensiblement sur l'ensemble de sa surface, soit une alternance de surfaces métalliques et de surfaces aimantées.

La paroi interne de l'arbre creux est avantageusement munie soit d'une ou de plusieurs surface(s) aimantée(s) coopérant avec une ou plusieurs surface(s) métallique(s) ou aimantée(s) de l'axe de rotation, soit d'une ou de plusieurs surface(s) métallique(s) coopérant avec une ou plusieurs surface(s) aimantée(s) de l'axe de rotation. De manière particulièrement avantageuse, lorsque l'axe de rotation comporte une surface métallique sensiblement sur l'ensemble de sa surface, la paroi interne de l'arbre creux présente au moins une surface aimantée coopérant avec cette surface métallique de l'axe de rotation ; ou bien lorsque l'axe de rotation comporte une surface aimantée de pôle A sensiblement sur l'ensemble de sa surface, la paroi interne de l'arbre creux présente au moins une surface métallique ou au moins une surface aimantée de pôle opposé au pôle A pour coopérer avec la surface aimantée de l'axe de rotation de manière à transmettre le mouvement de rotation lorsque le moyen d'entrainement est mis en rotation.

De préférence, ladite au moins une surface métallique ou ladite au moins une surface aimantée de la paroi interne de l'arbre creux est positionnée sur ou dans la paroi interne dudit arbre creux et ce, dans le prolongement de l'emplacement des ailettes.

Selon un mode de réalisation avantageux de l'invention, ledit au moins un moyen d'agitation comprend au moins une hélice présentant un moyeu et au moins une pale, et de préférence deux hélices. Avantageusement, ladite au moins une hélice comprend plusieurs pales de différentes formes possibles. Dans le cas où ledit au moins un moyen d'agitation présente plusieurs hélices, chacune des hélices peut comporter un nombre différent de pales, éventuellement de formes différentes.

Selon un mode de réalisation avantageux de l'invention, au moins une desdites ailettes est munie d'au moins un aimant coopérant avec au moins un contre-aimant positionné sur la surface inférieure dudit au moins un moyen d'agitation pour empêcher le frottement entre le moyen d'entrainement et ledit au moins un moyen d'agitation, ladite surface inférieure correspondant à la surface dudit au moins un moyen d'agitation opposée à la surface où ladite au moins une pale dudit au moins un moyen d'agitation fait saillie. De manière préférée, ledit au moins un aimant placé sur une ou plusieurs ailette(s) est, de préférence, positionné dans une région périphérique interne des ailettes, c'est-à-dire proche de l'axe de rotation de l'arbre creux, mais n'est pas situé sur ou dans la paroi de l'arbre creux.

Selon un autre mode de réalisation avantageux de l'invention, la turbine comprend en outre un corps fixe ayant sensiblement la forme d'un cylindre fermé, ledit corps fixe formant une enceinte dans laquelle les ailettes sont susceptibles de se déplacer.

Selon une variante de réalisation avantageuse de l'invention, le corps fixe de la turbine, sous forme de cylindre, est muni d'un ou de plusieurs aimant(s) dans la région périphérique externe de la surface du ou des disque(s) dudit cylindre. De manière particulièrement avantageuse, ce ou ces aimants a(ont) une polarité identique audit au moins un contre-aimant du moyen d'agitation afin d'empêcher le frottement entre le moyen d'entrainement et ledit au moins un moyen d'agitation. De préférence, ce ou ces aimants a(ont) également une polarité identique à l'aimant ou les aimants positionné(s) sur les ailettes pour repousser plus efficacement la surface inférieure dudit au moins un moyen d'agitation.

Selon un autre mode de réalisation avantageux de l'invention, le système d'agitation comprend en outre un moyen d'actionnement du moyen d'entrainement constitué par une arrivée de fluide ou d'électricité (charges électriques).

Selon une variante de réalisation avantageuse de l'invention, le fluide est un gaz choisi parmi le groupe constitué par l'air, l'azote, le dioxyde de carbone, l'argon, le dioxygène, et un mélange quelconque de ceux-ci.

Selon un mode de réalisation avantageux de l'invention, ladite arrivée de fluide sert également d'aérateur du milieu à mélanger dans la poche. L'utilisation du dioxyde de carbone, du dioxygène, de l'azote, de l'air ou d'un mélange de ces gaz en tant que fluide aérateur est avantageusement préférée puisque chacun de ces gaz peut servir d'élément de nutrition aux microorganismes en croissance.

Selon un autre mode de réalisation avantageux de l'invention, la hauteur dudit système d'agitation à usage unique dans la poche est variable et peut être variée pendant l'agitation.

L'invention telle que précédemment définie et telle que résultant de la description des figures ci-après représentant des modes de réalisation actuellement préférés de l'invention, permet de résoudre les problèmes techniques précédemment énoncés dans les buts de l'invention et permet donc de réaliser un bioréacteur et un système d'agitation à usage unique peu coûteux et de conception simple, utilisable à l'échelle industrielle et commerciale.

D'autres buts, caractéristiques et avantages de l'invention apparaîtront clairement à la lumière de la description explicative qui va suivre, faite en référence aux modes de réalisation actuellement préférés de l'invention, faisant partie intégrante de l'invention mais donnés simplement à titre d'illustration et qui ne sauraient en aucune façon limiter la portée de l'invention.

### DESCRIPTION DES FIGURES

- La figure 1 représente une vue en perspective du système d'agitation à usage unique selon la présente invention et selon un mode de réalisation actuellement préféré.
- La figure 2 représente une vue en coupe longitudinale du système d'agitation à usage unique selon la présente invention et selon un mode de réalisation actuellement préféré.
- La figure 3 représente une vue en perspective du bioréacteur jetable selon la présente invention et selon un premier mode de réalisation actuellement préféré.
- La figure 4 représente une vue en coupe longitudinale d'un moyen de fixation du système d'agitation à usage unique dans la poche flexible du bioréacteur jetable selon la présente invention et selon le premier mode de réalisation actuellement préféré.
- La figure 5 représente une vue en perspective du bioréacteur jetable selon la présente invention et selon un deuxième mode de réalisation actuellement préféré.
- La figure 6 représente une vue en coupe longitudinale d'un moyen de fixation du système d'agitation à usage unique dans la poche flexible du bioréacteur jetable selon la présente invention et selon le deuxième mode de réalisation actuellement préféré.

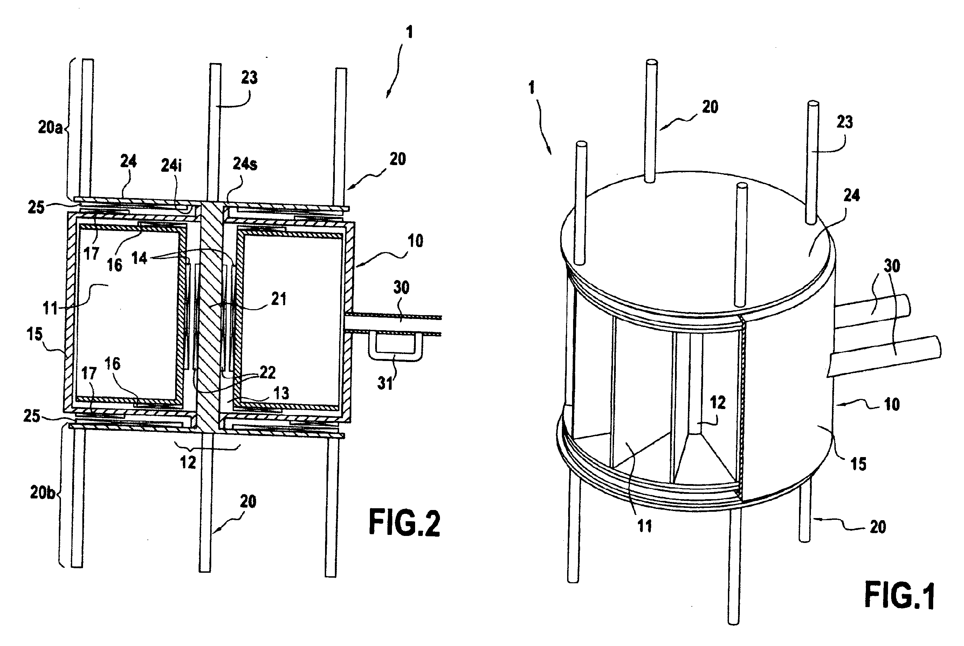

En référence aux figures 1 et 2, on a représenté un mode de réalisation actuellement préféré d'un système d'agitation à usage unique selon la présente invention.

Le système d'agitation 1 à usage unique comprend :
- un moyen d'entrainement 10, et
- un moyen d'agitation 20 entrainé en rotation par ledit moyen d'entrainement 10 via un couplage magnétique,
ledit moyen d'agitation 20 comprenant un axe de rotation 21. L'axe de rotation 21 présente au moins deux aimants 22. Le moyen d'entrainement 10 est une turbine 10 qui comprend des ailettes 11 faisant partie intégrante d'un arbre 12 creux. L'axe de rotation 21 du moyen d'agitation 20 est placé dans le creux 13 de l'arbre 12 et la paroi dudit arbre 12 creux présente dans le prolongement de chaque ailette 11 un aimant 14 qui attire un desdits au moins deux aimants 22 de l'axe de rotation 21 du moyen d'agitation 20. Les aimants 14 placés sur la paroi dudit arbre 12 creux et les aimants 22 de l'axe de rotation ont donc des polarités opposées pour s'attirer.

Comme illustrée aux figures 1 et 2, la turbine 10 comprend en outre un corps fixe 15 ayant sensiblement la forme d'un cylindre fermé 15, ledit corps fixe 15 formant une enceinte dans laquelle les ailettes 11 sont susceptibles de se déplacer.

Les figures 1 et 2 montrent que le moyen d'agitation 20 comprend deux hélices 20a, 20b présentant un moyeu 24 et quatre pales 23. La première hélice peut bien évidemment comporter un nombre différent de pales, éventuellement de formes différentes, par rapport à la deuxième hélice.

Les figures 1 et 2 montrent également que chaque ailette 11 est munie d'un aimant 16 coopérant avec un contre-aimant 25 positionné sur la surface inférieure 24i du moyen d'agitation 20 pour empêcher le frottement entre le moyen d'entrainement 10 et le moyen d'agitation 20, ladite surface inférieure 24i correspondant à la surface dudit au moins un moyen d'agitation opposée à la surface 24s où les pales 23 du moyen d'agitation 20 font saillie. L'aimant 16 placé sur l'ailette 11 est positionné dans une région périphérique interne des ailettes, c'est-à-dire proche de l'axe de rotation de l'arbre 12 creux, mais n'est pas situé sur ou dans la paroi de l'arbre creux.

Comme illustré aux figures 1 et 2, le corps fixe 15 de la turbine 10, sous forme de cylindre, est muni de plusieurs aimants 17 dans la région périphérique externe de la surface de chacun des disques dudit cylindre. Ces aimants 17 ont une polarité identique aux contre-aimants 25 du moyen d'agitation 20 afin d'empêcher le frottement entre le moyen d'entrainement 10 et ledit au moins un moyen d'agitation 20. En outre, ces aimants 17 ont également une polarité identique aux aimants 16 positionnés sur les ailettes 11 pour repousser plus efficacement la surface inférieure 24i dudit au moins un moyen d'agitation 20.

Les figures 1 à 6 montrent que le système d'agitation 20 comprend en outre un moyen d'actionnement 30 du moyen d'entrainement 10 constitué par une arrivée de fluide. Le fluide est un gaz pouvant être choisi parmi le groupe constitué par l'air, l'azote, le dioxyde de carbone, l'argon, le dioxygène, et un mélange quelconque de ceux-ci. L'arrivée du gaz sert également d'aérateur 31 du milieu à mélanger. Cette dernière configuration peut permettre au gaz utilisé de constituer un apport d'éléments de nutrition à des microorganismes en croissance.

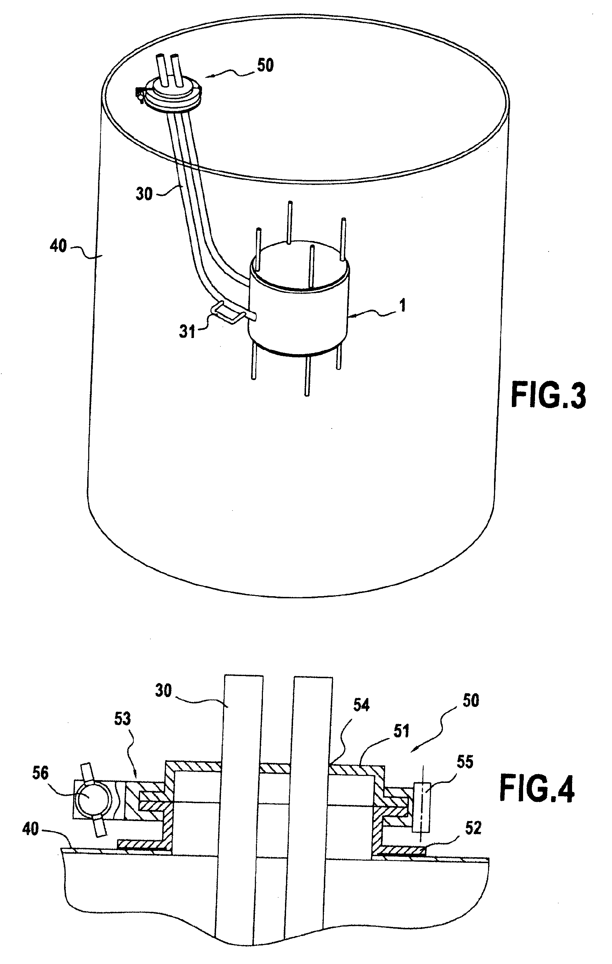

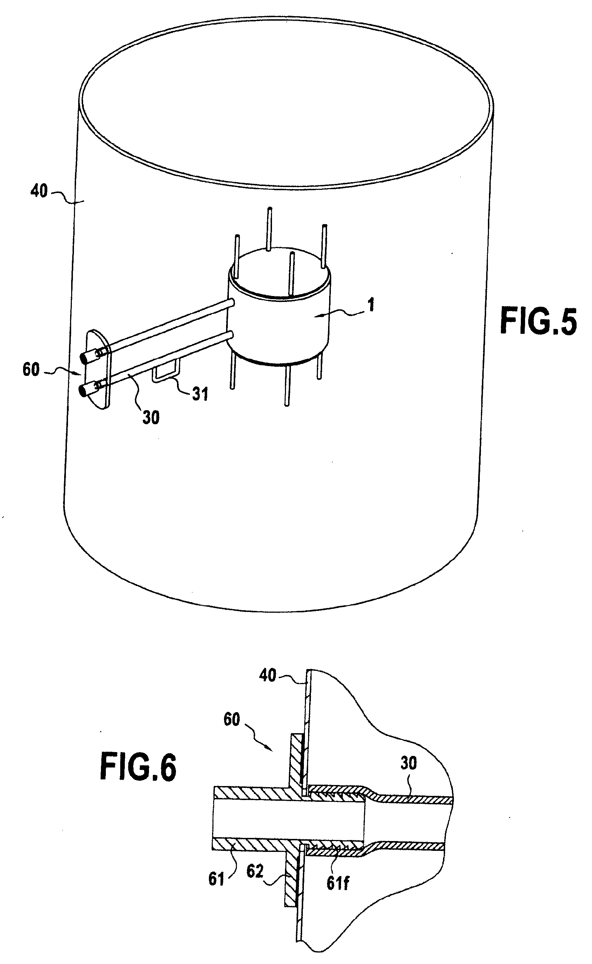

En référence aux figures 3 à 6, en particulier aux figures 3 et 5, on a représenté un premier et deuxième modes de réalisation actuellement préférés d'un bioréacteur jetable selon la présente invention.

Le bioréacteur jetable de chacun de ces modes de réalisation comprend une poche 40 flexible réalisée dans un matériau polymérique et un système d'agitation 1 à usage unique tel que précédemment décrit.

Comme représenté aux figures 3 à 6, le système d'agitation 1 à usage unique est maintenu de manière stabilisée à la poche 40 flexible du bioréacteur jetable à l'aide d'un moyen de fixation 50 ; 60. Ce moyen de fixation 50 ; 60 permet de modifier la hauteur du système d'agitation 1 à usage unique dans la poche 40 lorsque le système d'agitation 1 à usage unique est en cours d'utilisation ou lorsqu'il ne fonctionne pas.

Les figures 3 et 4 illustrent le moyen de fixation 50 selon le premier mode de réalisation actuellement préféré du bioréacteur jetable (raccord des tuyaux d'arrivée de gaz par le haut de la poche). Ledit moyen de fixation 50 est constitué d'une pièce mâle 51 et d'une pièce femelle 52 raccordées par un joint métallique 53. La pièce femelle 52 en matière plastique est soudée à la poche 40. La matière plastique peut avantageusement être choisie parmi le groupe constitué par le polyéthylène, le polypropylène, les polyamides, le polyétheréthercétone (PEEK) et le copolymère d'éthylène et d'alcool vinylique (EVOH). La pièce mâle 51 est munie de deux trous 54 dans lesquels les tuyaux 30 d'arrivée de gaz sont placés de manière sensiblement fixe (la hauteur des tuyaux peut toutefois être modifiée sous l'action d'une force physique), et est placée sur la pièce femelle 52. Le joint métallique 53 de raccordement, composé de deux demi-cercles pouvant pivoter selon un axe commun 55 et d'une fermeture à vis 56, permet d'unir les pièces femelle et mâle en un bloc, le bloc étant solidaire à la poche 40 flexible. Les tuyaux d'arrivée de gaz peuvent parfois être placés dans des barres de renforcement pour les rendre plus rigides et solides. Bien que les figures 3 et 4 ne le montrent pas, les tuyaux d'arrivée de gaz sont maintenus à un support fixe à l'aide d'un système pince-noix.

Les figures 5 et 6 illustrent le moyen de fixation 60 selon le deuxième mode de réalisation actuellement préféré du bioréacteur jetable (raccord des tuyaux d'arrivée de gaz par le côté de la poche). Ledit moyen de fixation 60 est constitué d'un raccord double pour tuyaux. Ce raccord a la forme de deux raccords pour tuyaux d'arrosage en série, c'est-à-dire qu'il est essentiellement constitué de deux pièces mâles de forme tubulaire 61 ayant chacun une extrémité filetée 61f. Ces pièces mâles comprennent en outre perpendiculairement à leur tube 61 une surface 62 sensiblement plane, en matière plastique, permettant au raccord double d'être soudé à la poche 40. La matière plastique de la surface 62 sensiblement plane peut avantageusement être choisie parmi le groupe constitué par le polyéthylène, le polypropylène, les polyamides, le polyétheréthercétone (PEEK) et le copolymère d'éthylène et d'alcool vinylique (EVOH). La forme filetée des extrémités 61f permet de connecter les tuyaux d'arrivée de gaz de manière étanche. Bien que les figures 5 et 6 ne le montrent pas, des barres de renforcement viennent se connecter aux extrémités non filetées et sont maintenus à un support fixe à l'aide d'un système pince-noix.

Ainsi, l'invention permet d'obtenir un bioréacteur jetable de conception simple, ainsi qu'un système d'agitation qui présente l'avantage de pouvoir s'adapter à n'importe quelle forme de bioréacteur jetable.

## Revendications

1. Bioréacteur jetable comprenant une poche (40) flexible réalisée dans un matériau polymérique, **caractérisé en ce qu'**il comprend en outre un système d'agitation (1) à usage unique placé en intégralité au sein de ladite poche (40) ; ledit système d'agitation (1) comprenant un moyen d'entraînement (10) et au moins un moyen d'agitation (20) entrainé en rotation par ledit moyen d'entraînement (10) via un couplage magnétique.

2. Bioréacteur selon la revendication 1, **caractérisé en ce que** le système d'agitation (1) comprend en outre un moyen d'actionnement (30) du moyen d'entraînement (10) constitué par une arrivée de fluide ou d'électricité (charges électriques).

3. Bioréacteur selon la revendication 2, **caractérisé en ce que** le fluide est un gaz choisi parmi le groupe constitué par l'air, l'azote, le dioxyde de carbone, l'argon, le dioxygène, et un mélange quelconque de ceux-ci.

4. Bioréacteur selon l'une quelconque des revendications 1 à 3, **caractérisé en ce que** ledit au moins un moyen d'agitation (20) comprend un axe de rotation (21), ledit axe de rotation (21) présentant au moins une surface métallique susceptible d'être attirée par une surface aimantée, de préférence un aimant, et/ou ledit axe de rotation présentant au moins une surface aimantée (22), de préférence au moins un aimant (22).

5. Bioréacteur selon l'une quelconque des revendications 1 à 4, **caractérisé en ce que** ledit au moins un moyen d'agitation (20) comprend au moins une hélice (20a, 20b) présentant un moyeu (24) et au moins une pale (23), et de préférence deux hélices (20a, 20b).

6. Bioréacteur selon la revendication 4 ou 5, **caractérisé en ce que** ledit moyen d'entrainement (10) est une turbine (10) qui comprend des ailettes (11) faisant partie intégrante d'un arbre (12) creux, ledit axe de rotation (21) dudit au moins un moyen d'agitation (20) étant placé dans ledit creux (13) de l'arbre et la paroi interne dudit arbre (12) creux présentant :
- au moins une surface métallique attirée par ladite surface aimantée de l'axe de rotation dudit au moins un moyen d'agitation ; et/ou
- au moins une surface aimantée (14), de préférence au moins un aimant (14), attirant ladite au moins une surface métallique ou aimantée (22) de l'axe de rotation (21) dudit au moins un moyen d'agitation (20).

7. Bioréacteur selon la revendication 6, **caractérisé en ce que** la turbine (10) comprend en outre un corps fixe (15) ayant sensiblement la forme d'un cylindre fermé (15), ledit corps fixe (15) formant une enceinte dans laquelle les ailettes (11) sont susceptibles de se déplacer.

8. Bioréacteur selon la revendication 6 ou 7, **caractérisé en ce qu'**au moins une desdites ailettes (11) est munie d'au moins un aimant (16) coopérant avec au moins un contre-aimant (25) positionné sur la surface inférieure (24i) dudit au moins un moyen d'agitation (20) pour empêcher le frottement entre le moyen d'entrainement (10) et ledit au moins un moyen d'agitation (20), ladite surface inférieure (24i) correspondant à la surface dudit au moins un moyen d'agitation (20) opposée à la surface (24s) où ladite au moins une pale (23) dudit au moins un moyen d'agitation (20) fait saillie.

9. Bioréacteur selon l'une quelconque des revendications 2 à 8, **caractérisé en ce que** ladite arrivée de fluide sert également d'aérateur (31) du milieu à mélanger dans la poche (40).

10. Bioréacteur selon l'une quelconque des revendications 1 à 9, **caractérisé en ce que** la hauteur dudit système d'agitation (1) à usage unique dans la poche (40) est variable et peut être variée pendant l'agitation.

## Patentansprüche

1. Einweg-Bioreaktor, umfassend einen aus einem Polymermaterial ausgebildeten flexiblen Beutel (40), **dadurch gekennzeichnet, daß** er ferner ein Einweg-Rührsystem (1), das vollständig innerhalb des Beutels (40) angeordnet ist, umfaßt, wobei das Rührsystem (1) ein Antriebsmittel (10) und wenigstens ein Rührmittel (20), das durch das Antriebsmittel (10) über eine magnetische Kupplung drehangetrieben wird, umfaßt.

2. Bioreaktor nach Anspruch 1, **dadurch gekennzeichnet, daß** das Rührsystem (1) ferner ein Mittel zum Betätigen (30) des Antriebsmittels (10) umfaßt, das durch eine Zuführung von Fluid oder Elektrizität (elektrische Ladungen) gebildet ist.

3. Bioreaktor nach Anspruch 2, **dadurch gekennzeichnet, daß** das Fluid ein Gas ist, das aus der Gruppe bestehend aus Luft, Stickstoff, Kohlendioxid, Argon, Disauerstoff und einer beliebigen Mischung dieser ausgewählt ist.

4. Bioreaktor nach einem der Ansprüche 1 bis 3, **dadurch gekennzeichnet, daß** das wenigstens eine Rührmittel (20) eine Rotationsachse (21) umfaßt, wobei die Rotationsachse (21) wenigstens eine Metallfläche aufweist, die geeignet ist, durch eine Magnetfläche, vorzugsweise einen Magnet, angezogen zu werden, und/oder wobei die Rotationsachse wenigstens einen Magnetfläche (22), vorzugsweise wenigstens einen Magnet (22) aufweist.

5. Bioreaktor nach einem der Ansprüche 1 bis 4, **dadurch gekennzeichnet, daß** das wenigstens eine Rührmittel (20) wenigstens einen Propeller (20a, 20b), der eine Nabe (24) und wenigstens ein Blatt (23) aufweist, und vorzugsweise zwei Propeller (20a, 20b) umfaßt.

6. Bioreaktor nach Anspruch 4 oder 5, **dadurch gekennzeichnet, daß** das Antriebsmittel (10) eine Turbine (10) ist, die Flügel (11), welche fester Bestandteil einer Hohlwelle (12) sind, umfaßt, wobei die Rotationachse (21) des wenigstens einen Rührmittels (20) in dem Hohlraum (13) der Welle angeordnet ist und wobei die Innenwand der Hohlwelle (12) aufweist:
- wenigstens eine Metallfläche, die durch die Magnetfläche der Rotationsachse des wenigstens einen Rührmittels angezogen wird, und/oder
- wenigstens ein Metallfläche (14), vorzugsweise wenigstens einen Magnet (14), der die wenigstens eine Metall- oder Magnetfläche (22) der Rotationsachse (21) des wenigstens einen Rührmittels (20) anzieht.

7. Bioreaktor nach Anspruch 6, **dadurch gekennzeichnet, daß** die Turbine (10) ferner einen festen Körper (15), der im wesentlichen die Form eines geschlossenen Zylinders (15) aufweist, umfaßt, wobei der feste Körper (15) einen Raum bildet, in dem sich die Flügel (11) bewegen können.

8. Bioreaktor nach Anspruch 6 oder 7, **dadurch gekennzeichnet, daß** wenigstens einer der Flügel (11) mit wenigstens einem Magnet (16) versehen ist, der mit wenigstens einem Gegenmagnet (25) zusammenwirkt, welcher an der unteren Fläche (24i) des wenigstens einen Rührmittels (20) angeordnet ist, um die Reibung zwischen dem Antriebsmittel (10) und dem wenigstens einen Rührmittel (20) zu verhindern, wobei die untere Fläche (24i) der Fläche des wenigstens einen Rührmittels (20) entspricht, die der Fläche (24s) gegenüberliegt, an der das wenigstens eine Blatt (23) des wenigstens einen Rührmittels (20) vorspringt.

9. Bioreaktor nach einem der Ansprüche 2 bis 8, **dadurch gekennzeichnet, daß** die Fluidzuführung auch als Belüfter (31) des in dem Beutel (40) zu mischenden Mediums dient.

10. Bioreaktor nach einem der Ansprüche 1 bis 9, **dadurch gekennzeichnet, daß** die Höhe des Einweg-Rührsystems (1) in dem Beutel (40) variabel ist und während des Rührens variiert werden kann.

## Claims

1. A disposable bioreactor including a flexible pouch (40) made of a polymer material, **characterized in that** it also includes a single-use stirring system (1) placed entirely within said pouch (40); said stirring system (1) comprising a drive means (10) and at least one stirring means (20) driven in rotation by said drive means (10) via magnetic coupling.

2. A bioreactor according to Claim 1, **characterized in that** the stirring system (1) also comprises a means (30) of actuating the drive means (10) consisting of a fluid or electricity (electrical charges) supply line.

3. A bioreactor according to Claim 2, **characterized in that** the fluid is a gas selected from the group consisting of air, nitrogen, carbon dioxide, argon, diatomic oxygen and any mixture thereof.

4. A bioreactor according to any one of Claims 1 to 3, **characterized in that** said at least one stirring means (20) comprises a rotation axle (21), said rotation axle (21) having at least one metal surface capable of being attracted by a magnetized surface, preferably a magnet, and/or said rotation axle having at least one magnetized surface (22), preferably at least one magnet.

5. A bioreactor according to any one of Claims 1 to 4, **characterized in that** said at least one stirring means (20) comprises at least one propeller (20a, 20b) having a hub (24) and at least one blade (23), and preferably two propellers (20a, 20b).

6. A bioreactor according to Claim 4 or 5, **characterized in that** said drive means (10) is a turbine (10) which includes vanes (11) forming an integral part of a hollow shaft (12), said rotation axle (21) of said at least one stirring means (20) being placed within said hollow (13) of the shaft and the inner wall of said hollow shaft (12) having:
- at least one metal surface attracted by said magnetized surface of the rotation axle of said at least one stirring means; and/or
- at least one magnetized surface (14), preferably at least one magnet (14), attracting said at least one metal or magnetized surface (22) of the rotation axle (21) of said at least one stirring means (20).

7. A bioreactor according to Claim 6, **characterized in that** the turbine (10) also comprises a fixed body (15) having substantially the shape of a closed cylinder (15), said fixed body (15) forming an enclosure wherein the vanes (11) are free to move.

8. A bioreactor according to Claim 6 or 7, **characterized in that** at least one of said vanes (11) is provided with at least one magnet (16) cooperating with at least one counter-magnet (25) positioned on the lower surface (24i) of said at least one stirring means (20) to prevent friction between the drive means (10) and said at least one stirring means (20), said lower surface (24i) corresponding to the surface of said at least one stirring means (20) opposite the surface (24s) from which said at least one vane (23) of said at least one stirring means (20) projects.

9. A bioreactor according to any one of Claims 2 to 8, **characterized in that** said fluid supply line also serves as an aerator (31) of the medium to be mixed within the pouch (40).

10. A bioreactor according to any one of Claims 1 to 9, **characterized in that** the height of said single-use stirring system (1) in the pouch (40) is variable and can be varied during stirring.
